# EUROPÄISCHE PATENTANMELDUNG

(11) **EP 0 602 442 A2**
(43) Veröffentlichungstag der Anmeldung: **22.06.1994**
(21) Anmeldenummer: 93119242.1
(22) Anmeldetag: 30.11.1993
(51) Int. Cl.: C07C 29/141, C07C 31/125, C07C 45/50, C07C 47/02

(54) **Verfahren zur Herstellung von höheren, vorwiegend unverzweigten, primären Alkoholen**

(30) Priorität: 17.12.1992 DE 4242725
(71) Anmelder: HOECHST AKTIENGESELLSCHAFT, D-65926 Frankfurt am Main (DE)
(72) Erfinder: Bahrmann, Helmut, Dr. Dipl.-Chem., D-46499 Hamminkeln (DE); Deckers, Gregor, Dr. Dipl.-Chem., D-46509 Xanten (DE); Greb, Wolfgang, Dr. Dipl.-Chem., D-46535 Dinslaken (DE); Heymanns, Peter, Dr. Dipl.-Chem., D-45147 Essen (DE); Lappe, Peter, Dr. Dipl.-Chem., D-46539 Dinslaken (DE); Müller, Thomas, Dipl.-Ing., D-46535 Dinslaken (DE); Szameitat, Jürgen, Dr. Dipl.-Chem., D-46487 Wesel (DE); Wiebus, Ernst, D-46147 Oberhausen (DE)

(57) **Zusammenfassung**

Zur Herstellung höherer, vorwiegend unverzweigter, primärer Alkohole werden Olefine aus der Fischer-Tropsch-Synthese in Gegenwart von Wasser sowie Rhodium in metallischer Form oder als Verbindung und einem wasserlöslichen Phosphin als Katalysator mit Wasserstoff und Kohlenmonoxid umgesetzt. Das erhaltene Reaktionsprodukt wird hydriert. Als wasserlösliche Phosphine werden Verbindungen eingesetzt, deren Anion ein mindestens einen sulfonierten oder carboxylierten, aromatischen Rest enthaltendes Phosphin und deren Kation ein quaternäres Ammonium- oder Phosphoniumion ist.

## Beschreibung

Die Erfindung betrifft ein Verfahren zur Herstellung von höheren, vorwiegend unverzweigten, primären Alkoholen durch Hydroformylierung von Olefinen aus der Fischer-Tropsch-Synthese in Gegenwart wasserlöslicher Rhodiumkomplexverbindungen als Katalysatoren.

Höhere, d.h. 6 bis 20 Kohlenstoffatome enthaltende unverzweigte primäre Alkohole finden als Zwischenprodukte in der chemischen Technik umfangreiche Anwendung. So werden aus C₈- bis C₁₂-Alkoholen durch Veresterung mit Phthalsäure Weichmacher hergestellt, die sich durch hohe Kälteflexibilität auszeichnen. Natriumsalze von n-Alkylsulfaten, gewonnen durch Veresterung von konzentrierter Schwefelsäure mit unverzweigten Fettalkoholen, werden biologisch abgebaut und haben daher als umweltverträgliche synthetische Waschmittel große Bedeutung erlangt. Ester auf Basis geradkettiger primärer Alkohole mit verzweigten Dicarbonsäuren sind wertvolle Schmiermittel, die insbesondere in Flugzeugmotoren angewendet werden.

Die vorstehend beispielhaft wiedergegebenen Einsatzmöglichkeiten für geradkettige primäre Alkohole erklären das große Interesse an Verfahren zur ihrer Herstellung, die von billigen Rohstoffen ausgehen und technisch möglichst einfach ausgeführt werden können. Hinzu kommt die Forderung, daß im Verlauf des Herstellungsverfahrens geradkettige Einsatzstoffe nicht isomerisiert und, sofern die Möglichkeit zur Bildung sekundärer und/oder tertiärer Alkohole neben primären Alkoholen gegeben ist, primäre Alkohole eindeutig bevorzugt werden.

Nach der DE-C-2 855 421 setzt man zur Herstellung von Dinonylphthalat-Weichmachern C₉-Alkohole ein, die durch Dimerisierung von Butenen in Gegenwart aluminiumorganischer Verbindungen als Katalysatoren zu einem Octengemisch und anschließende Hydroformylierung erhalten wurden.

Durch Hydroformylierung einer Butenfraktion, Aldolkondensation des erhaltenen Aldehydgemisches und anschließende Hydrierung gewinnt man C₁₀-Alkohole, die entsprechend dem Verfahren der EP-A-03 66 089 zu Phthalsäureestern weiterverarbeitet werden.

Ein anderer Weg zur Gewinnung von Di-decylphthalat-Gemischen ist in der EP-A-04 24 767 beschrieben. Die Herstellung der Ester erfolgt nach einem mehrstuf igen Verfahren durch Dimerisierung von Butengemischen, Hydroformylierung und Hydrierung des resultierenden Octengemisches zu einem Nonanolgemisch, Dehydratisierung des Nonanolgemisches unter Bildung eines Nonengemisches und Hydroformylierung und Hydrierung des Nonengemisches unter Bildung eines Decanolgemisches.

Die bekannten Verfahren werden noch nicht allen wirtschaftlichen und technischen Forderungen gerecht, die an einen in industriellem Maßstab ausgeübten Prozeß gestellt werden. So stehen die Ausgangsstoffe nicht immer in ausreichender Menge oder kostengünstig zur Verfügung oder ihre Umwandlung in die gewünschten geradkettigen Alkohole ist an aufwendige Prozesse gebunden.

Es bestand daher die Aufgabe ein Verfahren zu entwickeln, das von preiswerten Rohstoffen ausgeht und die gewünschten Alkohole auf einfachem Wege in hoher Ausbeute liefert.

Die Erfindung besteht in einem Verfahren zur Herstellung von höheren, vorwiegend unverzweigten, primären Alkoholen durch Umsetzung von Olefinen mit Kohlenmonoxid und Wasserstoff zu Aldehyden in flüssiger Phase bei Temperaturen von 20 bis 150°C und Drücken von 0,1 bis 20 MPa in Gegenwart von Wasser sowie Rhodium in metallischer Form oder als Verbindung und einem wasserlöslichen Phosphin als Katalysator und Hydrierung der Aldehyde zu Alkoholen. Es ist dadurch gekennzeichnet, daß durch Fischer-Tropsch-Synthese erhaltene Olefine mit 6 bis 20 Kohlenstoffatomen im Molekül oder Gemische aus 2 oder mehreren solcher Olefine in Gegenwart von wasserlöslichen Salzen umgesetzt werden, deren Anion ein, mindestens einen sulfonierten oder carboxylierten aromatischen Rest enthaltendes Phosphin ist, und deren Kation oder Kationen, entsprechend der Ladung des Anions, ein oder mehrere Ionen der allgemeinen Formel 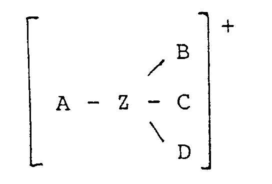
sind, wobei Z Stickstoff oder Phosphor bedeutet, A für einen Alkyl oder Aralkylrest mit 7 bis 18 Kohlenstoffatomen steht und B, C und D geradkettige oder verzweigte Alkylreste mit 1 bis 4 Kohlenstoffatomen sind.

Die als Ausgangsmaterial zur Herstellung von höheren, vorwiegend unverzweigten primären Alkoholen nach dem beanspruchten Verfahren verwendeten Olefine werden durch Fischer-Tropsch-Synthese erhalten. Unter dieser Bezeichnung versteht man die katalytische Kohlenoxid-Hydrierung an Feststoffkatalysatoren, die unter Aufbau aliphatischer Molekülketten verläuft. Als katalytisch wirksam haben sich die Metalle Eisen, Kobalt, Nickel und Ruthenium erwiesen, Bedeutung in der Praxis hat insbesondere Eisen erlangt. Für die technische Durchführung der Synthese (s. hierzu z.B. Ullmanns Encyclopädie der technischen Chemie 4. Auflage (1977), Band 14, Seiten 329 ff) wurden eine Reihe unterschiedlicher Verfahren entwickelt, von denen gegenwärtig zwei industriell genutzt werden. Im "Arge-Prozeß" werden Wasserstoff und Kohlenmonoxid in Festbettreaktoren bei einem Druck von 2,5 MPa und Temperaturen zwischen 220 und 225°C umgesetzt, der "Synthol-Prozeß" arbeitet mit Flugstaubreaktoren bei 2,3 MPa und 320 - 340°C. Die Fischer-Tropsch-Synthese verläuft, unabhängig vom angewandten Verfahren, nicht sehr selektiv, sondern liefert komplex zusammengesetzte Gemische, die gesättigte und ungesättigte Kohlenwasserstoffe unterschiedlicher Molekülgröße, Alkohole und daneben auch Aldehyde, Ketone und Säuren enthalten. Der Olefinanteil im Reaktionsprodukt kann bis zu etwa 70 % betragen, in der Hauptsache handelt es sich um unverzweigte Verbindungen mit 5 bis 18 Kohlenstoffatomen im Molekül und endständiger Doppelbindung. Die Bestandteile des Reaktionsgemisches werden auf konventionelle Weise durch Destillation voneinander getrennt. Ihr kann sich eine Feinreinigung anschließen, um einzelne Verbindungen in hoher Reinheit zu gewinnen. So können z.B. verzweigte Kohlenwasserstoffe von ihren geradkettigen Isomeren über Harnstoff-Einschlußverbindungen getrennt werden.

Die Hydroformylierung der, wie vorstehend beschrieben, erhaltenen Olefine muß unter Bedingungen erfolgen, die sicherstellen, daß geradkettige Aldehyde entstehen. Daher muß die Carbonylgruppe an das endständige Kohlenstoffatom des Olefinmoleküls gelenkt werden und überdies muß die Doppelbindung unter den Reaktionsbedingungen ihre Lage im Molekül beibehalten, d.h. sie darf nicht von der Endposition in das Innere der Molekülkette wandern. Als besonders geeignet, dieses Problem zu lösen, hat sich die Umsetzung der ungesättigten Verbindungen mit Kohlenmonoxid und Wasserstoff in einem flüssigen Zweiphasensystem erwiesen, das aus dem, gegebenenfalls in einem organischen Lösungsmittel gelösten, Olefin und sich bildendem Reaktionsprodukt als organische und der Rhodiumverbindung und wasserlösliches Phosphin enthaltenden Katalysatorlösung als wäßrige Phase besteht.

Als wasserlösliche Phosphine werden im Rahmen der erfindungsgemäßen Arbeitsweise Salze eingesetzt, deren Anion aus einem Phosphin gebildet wird, das mindestens einen sulfonierten oder carboxylierten aromatischen Rest enthält. Der Begriff Phosphin schließt auch solche Verbindungen des dreiwertigen Phosphors ein, in denen das Phosphoratom Bestandteil eines heterocyclischen Ringes ist. Der aromatische Rest kann unmittelbar oder über andere Gruppen an das Phosphoratom des Phosphins gebunden sein. Beispiele für aromatische Reste sind der Phenyl- und der Naphthylrest. Sie können einfach oder mehrfach sulfoniert oder carboxyliert und darüber hinaus durch weitere Atomgruppierungen oder Atome wie Alkyl, Hydroxyl, Halogen (insbesondere Fluor) substituiert sein. Außer Monophosphinen können auch Polyphosphine, insbesondere Diphosphine, die mindestens einen sulfonierten oder carboxylierten aromatischen Rest enthalten, das Anion bilden. Beispiele für geeignete Monophosphine sind die Triphenylphosphin-mono-, di- oder trisulfonate oder - carboxylate. Diphosphin-Anionen leiten sich bevorzugt von Biarylverbindungen der allgemeinen Formel 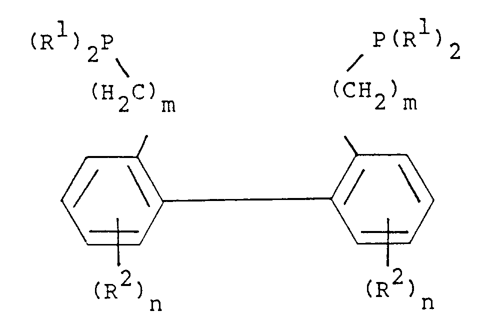
ab, die durch mindestens einen Sulfonat-(SO₃⁻)-Rest oder Carboxylat-(COO⁻)-Rest substituiert sind. In der allgemeinen Formel stehen R¹ für gleiche oder verschiedene Alkyl-, Cycloalkyl-, Phenyl-, Tolyl- oder Naphthylreste, R² ist gleich oder verschieden und bedeutet Wasserstoff, Alkyl- oder Alkoxyreste mit 1 bis 14 Kohlenstoffatomen, ferner Cycloalkyl-, Aryl- oder Aroxyreste mit 6 bis 14 Kohlenstoffatomen oder einen annelierten Benzolring, m ist gleich oder verschieden und eine ganze Zahl von 0 bis 5 und n ist ebenfalls gleich oder verschieden und steht für ganze Zahlen von 0 bis 4. Bewährte Vertreter dieser Verbindungsklasse sind die durch Sulfonierung von 2,2'-Bis(diphenylphosphinomethyl)-1,1'-biphenyl oder 2,2'-Bis(diphenylphosphinomethyl)-1,1'-binaphthyl erhaltenen Produkte. Als Beispiel des Anions einer heterocyclischen Phosphorverbindung ist das 3,4-Dimethyl-2,5,6-tris(p-sulfonatophenyl)-1-phosphanorbornadien zu nennen.

Als Kation enthalten die Salze entsprechend der Ladung des Anions mindestens ein Ion der allgemeinen Formel 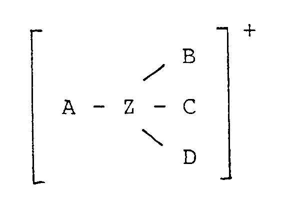
wobei Z Stickstoff oder Phosphor bedeutet, A für einen Alkyl- oder Aralkylrest mit 7 bis 18 Kohlenstoffatomen steht und B, C und D geradkettige oder verzweigte Alkylreste mit 1 bis 4 Kohlenstoffatomen sind.

Beispiele für Kationen der genannten Struktur, die sich zur Durchführung des neuen Verfahrens eignen, sind das Trimethylcetylammonium-, Trimethyldodecylammonium-, Tributyldodecylammonium-, Dodecylethyl-dimethylammonium-, Triethylbenzylammonium-, Trimethyltetradecylphosphonium-, Tributylhexadecylphosphoniumion.

Die nach dem neuen Verfahren als Katalysatorkomponente verwendeten Oniumverbindungen fördern die Löslichkeit des organischen Substrats in der wäßrigen Phase und tragen so zur Erhöhung des Umsatzes bei. Ihre äußerst geringe Löslichkeit in der organischen Phase hat zur Folge, daß sie selbst und die Metallkomponente des Katalysatorsystems nicht oder nur in vernachlässigbar kleiner Menge zusammen mit dem Reaktionsprodukt aus der Reaktionszone ausgetragen werden. Ein gesonderter Aufarbeitungsschritt zur Rückgewinnung von Rhodium aus dem Aldehyd erübrigt sich daher.

Von besonderer Bedeutung ist, daß bei Verwendung der wasserlöslichen Phosphine nach der beanspruchten Arbeitsweise die erfindungsgemäß eingesetzten Olefine mit hoher Aktivität und Selektivität hydroformyliert werden, wobei die Struktur der umgesetzten Kohlenwasserstoffe erhalten bleibt, d.h. eine Isomerisierung nicht oder in nur unerheblichem Umfang auftritt. Überdies bewirken die Phosphine, daß nahezu ausschließlich endständige Olefine hydroformyliert werden. Der dadurch erzielte Trenneffekt hat eine weitere Reduzierung des Anteils verzweigter Verbindungen im Endprodukt zur Folge.

Die Herstellung der in dem beanspruchten Verfahren verwendeten Salze ist bekannt. Man geht von sulfonierte oder carboxylierte Arylreste enthaltenden Phosphinen oder Phosphorheterocyclen aus. Die Einführung des Sulfonatrestes erfolgt nach bewährten Verfahren durch Behandlung der Ausgangsverbindungen mit Oleum. Durch Variation der Reaktionsbedingungen, insbesondere der Reaktionszeit, der Reaktionstemperatur und des Verhältnisses von Phosphorverbindung zu Schwefeltrioxid kann der Sulfonierungsgrad der Ausgangsverbindungen gesteuert werden.

Die carboxylierten Phosphorverbindungen können auf verschiedenen Wegen, z.B. durch Umsetzung der Grundkörper mit Carbonylhalogeniden und insbesondere über Grignardverbindungen durch Reaktion mit Kohlendioxid erhalten werden.

Zweckmäßig gewinnt man aus dem Reaktionsprodukt zunächst in Wasser unlösliche, in organischen Lösungsmitteln jedoch lösliche Aminsalze. Sie werden anschließend durch Behandlung mit einem quartären Ammonium- bzw. Phosphoniumhydroxid in das gewünschte Onium-Salz des sulfonierten bzw. carboxylierten Triarylphosphins überführt.

Die Umsetzung der Olefine mit Wasserstoff und Kohlenmonoxid nach dem neuen Verfahren erfolgt bei Temperaturen von 20 bis 150°C, insbesondere 50 bis 120°C und Drücken von 0,1 bis 20 MPa, insbesondere 1 bis 10 MPa.

Der Katalysator kann dem Reaktionssystem präformiert zugesetzt werden. Mit gleich gutem Erfolg läßt er sich aber auch aus den Komponenten Rhodium oder Rhodiumverbindung und der wäßrigen Lösung des quartären Ammonium- bzw. Phosphoniumsalzes des sulfonierte oder carboxylierte aromatische Reste enthaltenden Phosphins unter Reaktionsbedingungen im Reaktionsgemisch, also in Gegenwart des Olefins, herstellen. Außer metallischem Rhodium in feinverteilter Form können als Rhodiumquelle wasserlösliche Rhodiumsalze wie Rhodiumchlorid, Rhodiumsulfat, Rhodiumacetat oder in organischen Medien lösliche Verbindungen wie Rhodium-2-ethylhexanoat oder unlösliche Verbindungen wie Rhodiumoxide eingesetzt werden.

Die Rhodiumkonzentration in der wäßrigen Katalysatorlösung beträgt 10 bis 2000 Gew.-ppm, bezogen auf die Lösung. Das quartäre Ammonium- bzw. Phosphoniumsalz des sulfonierten oder carboxylierten Phosphins wird in einer solchen Menge eingesetzt, daß auf 1 mol Rhodium 1 bis 1000 mol, vorzugsweise 2 bis 300 mol, Phosphinverbindung kommen.

Der pH-Wert der wäßrigen Katalysatorlösung soll nicht unter 2 liegen. Allgemein stellt man einen pH-Wert von 2 bis 13, vorzugsweise 4 bis 10, ein.

Die Zusammensetzung des Synthesegases, d.h. das Verhältnis von Kohlenmonoxid zu Wasserstoff, kann in weiten Grenzen variiert werden. Im allgemeinen setzt man ein Synthesegas ein, in dem das Volumenverhältnis von Kohlenmonoxid zu Wasserstoff 1 : 1 beträgt oder von diesem Wert nur wenig abweicht.

Die Umsetzung kann sowohl absatzweise als auch kontinuierlich durchgeführt werden.

Das Hydroformylierungsgemisch wird von der Katalysatorlösung durch einfache Phasentrennung abgeschieden. Es kann ohne weitere Vorbehandlung hydriert werden. Jedoch ist es auch möglich, zuvor die Aldehyde von begleitenden Nebenprodukten abzudestillieren. Die Wasserstoffanlagerung erfolgt in bekannter Weise in Gegenwart von Katalysatoren. Geeignet sind z.B. Hydrierkatalysatoren auf Basis von Nickel, Chrom oder Kupfer. Üblicherweise liegt die Hydriertemperatur zwischen 100 und 180°C und der Druck zwischen 1 und 10 MPa. Zur Reinigung werden die Alkohole destilliert. Sie eignen sich vorzüglich als Alkoholkomponente in Phthalsäureestern, die als Weichmacher verwendet werden sollen, sowie zur Herstellung von Alkylsulfonaten und Esterschmiermitteln.

Das folgende Beispiel erläutert das erfindungsgemäße Verfahren, beschränkt es jedoch nicht.

### Beispiel

### Versuch 1: Hydroformylierung

In einem l l-Autoklav werden
185 g eines überwiegend n-Nonene enthaltenden Olefingemischs aus der Fischer-Tropsch-Synthese, sowie, als Katalysator,
550 g einer wäßrigen Lösung, die 66 mol-% Trinatrium-tri(m-sulfophenyl)phosphin und 33 mol-% Tri(trimethyltetradecyl)ammonium-tri(m-sulfophenyl)phosphin enthält, zusammen mit
5,44 ml Rhodiumacetat-Lösung (Rhodium-Konzentration: 20,21 g Rh/l), entsprechend einem Gehalt der vereinigten Lösungen von 80 mmol P(III) und 200 Gew.-ppm Rh
und zur Einstellung eines pH-Wertes von etwa 6
27,5 g einer Pufferlösung aus 10 mol Na-acetat und 1 mol Eisessig/l Lösung
vorgelegt. Der Autoklav wird zur Entfernung von Sauerstoff mehrfach mit Stickstoff gespült. Darauf leitet man in das Gemisch bei einem Druck von 2,5 MPa und 125°C unter Rühren Synthesegas (CO/H₂-Vol.-verhältnis = 1 : 1) ein. Die Reaktion wird nach 6 h abgebrochen, der Autoklav entspannt, die organische Phase (das Reaktionsprodukt) über einen Tauchstutzen entnommen und analysiert. Der Olefinumsatz beträgt 85 %, er bleibt bei mehrfacher Wiederholung der Reaktion mit derselben Katalysatorlösung unverändert.

### Versuch 2: Hydrierung

Das nach Versuch 1 erhaltene Rohprodukt wird in einem Autoklav in Gegenwart von 2 Gew.-% (bezogen auf das Rohprodukt) eines handelsüblichen Nickelkatalysators (60 Gew.-% Ni auf Kieselgur als Träger) bei 8 MPa und 150°C 3 h mit Wasserstoff hydriert. Nach Beendigung der Reaktion wird das Reaktionsgefäß abgekühlt, entspannt und das Reaktionsprodukt vom Katalysator abgetrennt.

### Versuch 3: Reinigung des Rohalkohols

Der Rohalkohol aus Versuch 2 wird in einer 1 m-Kolonne, die mit 3 mm Wendeln aus VA-Stahl gefüllt ist, destilliert. Bei einer Kopftemperatur von 93°C und 6,7 kPa Druck wird ein Vorlauf, bei 145°C und 6,7 kPa Druck ein Zwischenlauf abgenommen. Die Hauptfraktion (26 Gew.-% des eingesetzten Rohalkohols) erhält man bei 147°C Kopftemperatur und 6,7 kPa Druck. Der Alkoholgehalt im Rohprodukt beträgt 99,7 %.

### Versuch 4: Veresterung des Reinalkohols

2,3 mol des Isodecanols aus Versuch 3 wird mit 1 mol Phthalsäureanhydrid in Gegenwart von 6 mmol konzentrierter Schwefelsäure bei 135°C umgesetzt unter gleichzeitiger Entfernung des Reaktionswassers als Azeotrop mit Cyclohexan. Nach Beendigung der Umsetzung wird die Schwefelsäure mit 5 %iger Natronlauge neutralisiert und überschüssiger Alkohol bei 135°C und 1,5 kPa durch Wasserdampfdestillation abgetrennt. Der Rückstand wird mit Stickstoff bei 135°C und 2 bis 3 kPa Druck getrocknet und anschließend filtriert. Man erhält den Ester als klare Flüssigkeit.

Gegenüber handelsüblichen Esterweichmachern, deren Viscosität (bei 20°C) 120 bis 130 mPa.s beträgt, zeichnet sich der mit dem erfindungsgemäßen Alkohol hergestellte Weichmacher durch die sehr niedrige Viscosität von 64,8 mPa.s (bei 20°C) aus, ein Zeichen für die hohe Linearität des verwendeten Alkohols.

## Patentansprüche

1. Verfahren zur Herstellung höherer, vorwiegend unverzweigter, primärer Alkohole durch Umsetzung von Olefinen mit Kohlenmonoxid und Wasserstoff zu Aldehyden in flüssiger Phase bei Temperaturen von 20 bis 150°C und Drücken von 0,1 bis 20 MPa in Gegenwart von Wasser sowie Rhodium in metallischer Form oder als Verbindung und einem wasserlöslichen Phosphin als Katalysator und Hydrierung der Aldehyde zu Alkoholen, dadurch gekennzeichnet, daß durch Fischer-Tropsch-Synthese erhaltene Olefine mit 6 bis 20 Kohlenstoffatomen im Molekül oder Gemische aus 2 oder mehreren solcher Olefine in Gegenwart von wasserlöslichen Salzen umgesetzt werden, deren Anion ein, mindestens einen sulfonierten oder carboxylierten aromatischen Rest enthaltendes Phosphin ist, und deren Kation oder Kationen, entsprechend der Ladung des Anions, ein oder mehrere Ionen der allgemeinen Formel 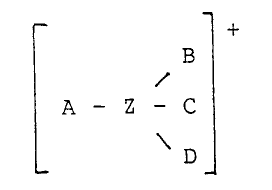 sind, wobei Z Stickstoff oder Phosphor bedeutet, A für einen Alkyl oder Aralkylrest mit 7 bis 18 Kohlenstoffatomen steht und B, C und D geradkettige oder verzweigte Alkylreste mit 1 bis 4 Kohlenstoffatomen sind.

2. Verfahren nach Anspruch 1, dadurch gekennzeichnet, daß sich das Anion des wasserlöslichen Salzes von einem mindestens einen sulfonierten oder carboxylierten aromatischen Rest enthaltenden Monophosphin ableitet.

3. Verfahren nach Anspruch 1, dadurch gekennzeichnet, daß sich das Anion des wasserlöslichen Salzes von einem mindestens einen sulfonierten oder carboxylierten aromatischen Rest enthaltenden Diphosphin ableitet.

4. Verfahren nach einem oder mehreren der Ansprüche 1 bis 3, dadurch gekennzeichnet, daß die sulfonierten oder carboxylierten aromatischen Reste im Anion Phenyl- oder Naphthylreste sind.

5. Verfahren nach einem oder mehreren der Ansprüche 1 bis 4, dadurch gekennzeichnet, daß die sulfonierten oder carboxylierten aromatischen Reste durch weitere Atomgruppierungen oder Atome, insbesondere Fluor, substituiert sind.

6. Verfahren nach einem oder mehreren der Ansprüche 1, 2 und 4, dadurch gekennzeichnet, daß die Anionen Triphenylphosphin-mono-, di- oder trisulfonate oder -carboxylate sind.

7. Verfahren nach einem oder mehreren der Ansprüche 1, 3, 4 und 5, dadurch gekennzeichnet, daß die Anionen durch mindestens einen Sulfonat- oder Carboxylatrest substituierte Biarylverbindungen der allgemeinen Formel 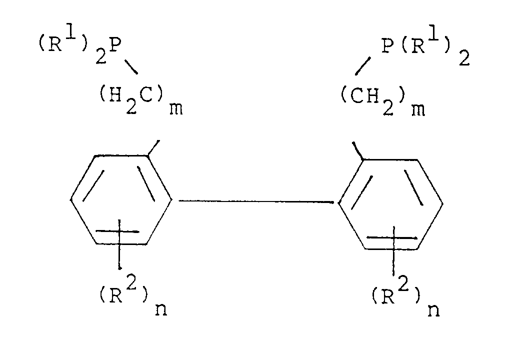 sind, in der R¹ für gleiche oder verschiedene Alkyl-, Cycloalkyl-, Phenyl-, Tolyl- oder Naphthylreste steht, R² gleich oder verschieden ist und Wasserstoff, Alkyl- oder Alkoxyreste mit 1 bis 14 Kohlenstoffatomen, ferner Cycloalkyl-, Aryl- oder Aroxyreste mit 6 bis 14 Kohlenstoffatomen oder einen annelierten Benzolring bedeutet, m gleich oder verschieden ist und für eine ganze Zahl von 0 bis 5 und n ebenfalls gleich oder verschieden ist und für eine ganze Zahl von 0 bis 4 steht.

8. Verfahren nach Anspruch 7, dadurch gekennzeichnet, daß das Anion durch Sulfonierung von 2,2'-Bis(diphenylphosphinomethyl)-1,1'-biphenyl oder 2,2'-Bis(diphenylphosphinomethyl)-1,1'-binaphthyl erhalten wird.

9. Verfahren nach einem oder mehreren der Ansprüche 1, 2 und 4, dadurch gekennzeichnet, daß das Anion das 3,4-Dimethyl-2,5,6-tris(p-sulfonatophenyl)-1-phosphanorbornadien ist.

10. Verfahren nach einem oder mehreren der Ansprüche 1 bis 9, dadurch gekennzeichnet, daß das Kation ein Trimethylcetylammonium-, Trimethyldodecylammonium-, Tributyldodecylammonium-, Dodecylethyl-dimethylammonium-, Triethylbenzylammonium-, Trimethyltetradecylphosphonium- oder ein Tributylhexadecylphosphoniumion ist.
